# EUROPEAN PATENT SPECIFICATION

(11) **EP 4 393 539 B1**
(45) Date of publication and mention of the grant of the patent: **28.05.2025**
(21) Application number: 23214649.8
(22) Date of filing: 06.12.2023
(51) Int. Cl.: A61N 1/04, A61N 1/18, A61N 1/36, A61H 7/00

(54) **SYSTEMS INCLUDING HANDHELD DEVICES TO DELIVER MICROCURRENTS AND/OR KINESIOLOGICAL SCULPTING, USEFUL IN SKIN CARE**
SYSTEME MIT TRAGBAREN VORRICHTUNGEN ZUR ABGABE VON MIKROSTRÖMEN UND/ODER KINESIOLOGISCHER UMFORMUNG ZUR HAUTPFLEGE
SYSTÈMES COMPRENANT DES DISPOSITIFS PORTATIFS POUR DÉLIVRER UN SCULPTAGE MICROCOURANT ET/OU KINÉSIOLOGIQUE, UTILES DANS LES SOINS DE LA PEAU

(30) Priority: 29.12.2022 US 202263576441 P; 01.06.2023 US 202363470342 P; 12.10.2023 US 202318379303
(43) Date of publication of application: 03.07.2024
(73) Proprietor: Micro Current Technology, Inc., Seattle, WA 98134 (US)
(72) Inventor: Suzuki, David, Seattle, WA 98134 (US)
(74) Representative: Gray, James

(56) References cited:
- EP-A1- 3 560 474
- WO-A1-2014/189096
- JP-B2- 6 344 992
- US-A1- 2022 354 732

## Description

### Field

This disclosure generally relates to skin care and cosmetic treatments, and in particular to systems including handheld devices and treatments that deliver microcurrents via skin tissue and muscle and/or which are also able to provide kinesiological sculpting via stretching or tensioning and/or squeezing or pinching bodily tissue (e.g., skin and muscles including tendons) for instance via lengthening and/or shortening of bodily tissue.

### BACKGROUND

### Description of the Related Art

Microcurrent technology for cosmetic use is based on the same mechanism of action as that of medical microcurrent, that is triggering a massive uptick of adenosine triphosphate (ATP) by way of stimulation of mitochondria. As with medical microcurrent, the availability of up to 500% more ATP expedites the healing properties for wounded or injured tissue and muscles, and also helps to maximize the condition of existing tissue and muscles. As people age they experience a significant reduction of collagen and elastin, particularly in the face and neck, which leads to fine lines, wrinkles, and sagging skin. People also experience atrophy in the muscles which further deteriorates their aesthetic visual appearance. This process of aging is directly related to the depreciation of mitochondria activity that ultimately reduces the availability of ATP to the skin and muscles, for example of the face and neck. Age typically drives the body's inability to keep up on the maintenance requirements in real time due to the lack of ATP. The damage that aggregates is what we perceive as the visual effects of aging. US2022354732 describes a rejuvenating beauty care tool for skin and body that enables the user to treat different skin and body areas using different therapeutic and cosmetic techniques. The apparatus may include a handle, a first arm, a second arm, a plurality of first rollers, and a plurality of second rollers.

### BRIEF SUMMARY

### Summary of the invention

The present invention is directed to a system according to claim 1. Additional features and embodiments of the invention are defined in the dependent claims.

### Summary of the disclosure

Applicant pioneered a microcurrent technology used to provide cosmetic services, and provided under the tradename Bio-Therapeutic. For example, Bio-Therapeutic offers two microcurrent facial toning systems, namely the Bio-Ultimate^{®} Platinum microcurrent facial toning system and the bt-nano^{®} microcurrent facial toning system. Each of the microcurrent facial toning systems includes a console and two ergonomic hand-held probes (*i.e.,* Azul^{™} ergonomic hand-held probes). In use, one of the hand-held probes is held in each hand of the service provider and used to apply microcurrents to bodily tissue (*e.g.,* facial skin tissue) in performing cosmetic services. The cosmetic service typically comprises two distinct operations. The first operation is referred to as sculpting and the second operation is referred to as skin work.

Applying the specified micro amperage, frequencies, and wave shapes to a portion of the body (*e.g.,* face), can advantageously stimulate the mitochondria, thereby increasing ATP. Applicant has determined that various movements (*e.g.,* sculpting movements) of the handheld probes or electrodes thereof on the bodily tissue (*e.g.,* skin tissue and/or underlying muscle and/or tendons of the face) can advantageously change or improve muscle tonicity (*e.g.,* tonicity of the muscle and/or tendons underlying the skin tissue), which in turn changes the visual appearance and contour of the particular portion of the body (*e.g.,* face) receiving the cosmetic service. The difference could be compared to the effect that one sees on a body after a good physical work out. With weightlifting, the muscles of the body strain, eventually creating micro tears in the muscles. As a defense, the muscles contract. As the muscles heal, they become healthier and stronger. If weightlifting is continued consistently, the body is on a continuous circle of evolution: tearing, contracting, healing, and becoming stronger. While the micro tears are by definition damage, there is no disruption of the mitochondria activity or the Krebs cycle.

To be clear, application of microcurrents differs significantly from electrical muscle stimulation (EMS) technology. EMS typically involves the delivery of currents in the range of 500 µA to 5,000 µA. In contrast, the microcurrents used in Bio-Therapeutic^{®} technology are typically equal to or less than 400 µA. The relatively higher amperage associated with EMS is detrimental to the mitochondria and the Krebs cycle, and tends to halt the production of ATP. EMS typically involves sufficient energy to cause a visual and physical contraction of the muscle resulting from the application of electrical current. In short, the amperage is sufficient to make the muscle jump and twitch. The body immediately recognizes this energy as a damaging, and contracts the muscles to stop the muscles from twitching. The high level of energy associated with EMS confuses the mitochondria (Krebs cycle) and the muscles, causing tissue use what energy they have left to defend the body by contracting the muscles. A consumer may initially perceive the contraction as a good result. However, when the ATP stores are depleted after a few days, the muscles become lax. In response, the consumer will often jolt the muscles again, shocking the muscles back into contraction. The laxity of the muscles now returns more swiftly. By the third or fourth time that the muscles are shocked, the muscles are now completely devoid of ATP and have no ability to move (*e.g.,* contract). The muscles are now flaccid. In addition to the muscles being depleted of ATP, the other bodily tissue has also been depleted of ATP. This quickly expedites the perceived aging process as the collagen and elastin in the skin tissue breaks down.

True microcurrent signals (≤ 400 µA) advantageously do not have enough energy to cause a visual or physical contraction of the muscle due to the electrical current. The microcurrents advantageously provide enough energy to benefit mitochondria activity, producing upwards of 500% more ATP cellular energy, without triggering muscle contraction.

The first operation (*i.e.,* sculpting) can include performing facial sculpting movements to physically lengthen and shorten muscles as a mechanism of action (kinesiology). Similar in some respect to massage, the bodily tissue can be physically manipulated to move the muscles into shorter or longer positions or states and the muscles react instantly. This is why a 45 minute massage can relax (lengthen) muscles and provide an amazing sense of relief, increased range of motion, and increased flexibility. Applicant has realized that similar techniques can be applied, for example to the face, however, focusing on shortening most muscles (*e.g.,* facial muscles, tendons) as those muscle typically have become elongated over time. The muscles (including tendons) react to the manipulation, and the increased levels of ATP induced by the delivery of microcurrent signals give the muscles the endurance to stay in this new position or state for a longer period of time - than might otherwise be achieved without the microamperage stimulation. Repeating the movements 2 to 3 days later accompanied by the delivery of microcurrent signals reinforces the new contour of the muscles, and continues to boost ATP levels, strongly encouraging the new contour as well as simultaneously energizing collagen and elastin synthesis.

The second operation (*i.e.,* skin work) focuses on stimulation of the mitochondria and circulatory benefits, *e.g.,* gliding the electrodes around the face and neck.

Bodily tissue is responsive to specific frequencies or ranges of frequencies and hence to specific signaling mechanisms. Applicant has developed technology that employs microcurrent signals with specific wave shapes that act as a delivery mechanism of specific frequencies to different depths of muscles including tendons, and other bodily tissue (*e.g.,* skin). Certain frequencies are particularly effective for cellular activation.

Although Applicant has established a robust catalog of successful frequencies throughout many years of experience, such does not change the fact that every person is frequency unique. Thus, Applicant has developed a technology that increases the total number of different microcurrent signals delivered during use.

Applicant's two microcurrent facial toning systems include two separate handheld probes. One of the handheld probes represents channel 1 and channel 2 +. The other of the handheld probes represents channel 1 and channel 2 -. The microcurrent signal employs a modulated biphasic square wave.

Thus, there are two fully independent channels, each with their own frequency. Applicant's unique approach also employs interferential channels, which allows for two independent channels to operate with completely different frequencies from the primary channels. The two frequencies collaborate when run in parallel with one another, creating two additional frequencies, for a total of four frequencies. This approach provides four (4) times the ability to achieve a desired result (*e.g.,* by increasing the likelihood of at least approximately matching the frequencies of any given person) and makes Applicant's technology more successful, consistently, because of this larger range of coverage.

Most handheld technology on the market is effectively EMS. EMS gains what limited results it can achieve by shocking the nerve motor points. These EMS devices typically work with two electrodes that are spatially fixed relative to one another, and have no ability to perform anything besides shocking the muscles into twitching. These EMS devices are generally not capable of realizing kinesiological movements.

As previously noted, Applicant's own micro current systems employ a console and two separate handheld probes to apply microcurrents during the cosmetic treatments.

In operation, the microcurrent flows into the skin from an electrode on one probe, does its work in the skin and muscles (including tendons), and then returns back via an electrode on the other probe. Applicant has realized that it would be advantageous to be able to keep the electrodes of the two handheld probes within a range of approximately 2.5 cm (one inch) to approximately 5cm (two (1-2) inches) of each other. To do so, one could attempt to place both electrodes on a single handheld probe. However, a single handle probe would make it difficult, if not impossible, to perform the desired sculpting movements.

Described herein are systems that combine two pairs of electrodes into one handheld device, while maintaining two independent channels and interferential technology. At least one of the pairs of electrodes is moveable with respect to a body of the handheld device and with respect to the other pair of electrodes, facilitating use for full kinesiology sculpting (*e.g.,* facial sculpting), for example moveable within a defined range of motion (*e.g.,* defined range of rotation or spacing). Such allows scooping and grabbing the muscle (including tendons) with one pair of electrodes (*e.g.,* a lower pair of electrodes) and then holding the tissue with the other pair of electrodes (*e.g.,* an upper pair of electrodes), while maintaining the electrodes of one pair within a defined range of distance from the electrodes of the other pair. Not only does such allow one handed operation, but such can achieve other advantages. For example, an amount of tension or force can be controlled via a biasing mechanism (*e.g.,* spring). A range of spacing or angle between electrodes can be controlled, for example having a minimum spacing or angle and/or a maximum spacing or angle. Such can advantageously ensure that the electrodes are within a specified distance or angle from one another to ensure a desired or specified delivery of microcurrent signals.

The electrodes are electrically conductive. The electrodes can comprise a material (*e.g.,* stainless steel) that can advantageously have a relative low amount of nickel or even be nickel free (*e.g.,* 420 stainless steel). The electrodes can advantageously have a rough outer surface.

The electrodes can be supported by respective arms. One pair of arms, or both pair of arms, can be biased toward a default configuration. For example, the arms can be biased via one or more tension springs, with or without a respective lever. The handheld device can include one or more stops to advantageously limit an amount of travel of the movable arms and hence of the electrodes carried by those moveable arms.

### BRIEF DESCRIPTION OF THE SEVERAL VIEWS OF THE DRAWINGS

In the drawings, identical reference numbers identify similar elements or acts. The sizes and relative positions of elements in the drawings are not necessarily drawn to scale. For example, the shapes of various elements and angles are not necessarily drawn to scale, and some of these elements may be arbitrarily enlarged and positioned to improve drawing legibility. Further, the particular shapes of the elements as drawn, are not necessarily intended to convey any information regarding the actual shape of the particular elements, and may have been solely selected for ease of recognition in the drawings.
Figure 1A is a top plan view of a system comprising a handheld device with integral circuitry, the system operable to deliver microcurrents via skin tissue and muscle, including tendons, and/or to provide kinesiological sculpting, according to one illustrated implementation.
Figure 1B is a front elevational view of the handheld device of Figure 1A.
Figure 1C is a rear elevational view of the handheld device of Figure 1A.
Figure 1D is an isometric view of the handheld device of Figure 1A.
Figure 1E is a right side elevational view of the handheld device of Figure 1A showing a second pair of arms in an un-rotated orientation or configuration relative to a first pair of arms.
Figure 1F is a left side elevational view of the handheld device of Figure 1A showing the second pair of arms in an un-rotated orientation or configuration relative to the first pair of arms.
Figure 1G is a left side elevational view of the handheld device of Figure 1A showing the second pair of arms in a fully rotated orientation or configuration relative to the first pair of arms.
Figure 2A is a cross-sectional view of the handheld device of Figure 1A showing the second pair of arms in the un-rotated orientation or configuration relative to the first pair of arms.
Figure 2B is a cross-sectional view of the handheld device of Figure 1A showing the second pair of arms in the fully rotated orientation or configuration relative to the first pair of arms.
Figure 3A is a first exploded view of the handheld device of Figure 1A, according to one illustrated implementation.
Figure 3B is a second exploded view of the handheld device of Figure 1A, according to one illustrated implementation.
Figure 4A is a schematic diagram showing a portion of circuitry operable to generate microcurrents for delivery via skin tissue and muscle, including tendons, which can be employed with the handheld device of Figure 1A, according to one illustrated implementation.
Figure 4B is a schematic diagram showing a portion of circuitry of Figure 1A, according to one illustrated implementation.
Figure 4C is a schematic diagram showing a portion of circuitry of Figure 1A, according to one illustrated implementation.
Figure 5 is a schematic diagram showing a system comprising a console and a handheld device communicatively coupled to the console, the console comprising circuitry to generate microcurrents and the handheld device including two pairs of arms with electrodes, at least one pair of arms moveable within a defined range of motion with respect to the other pair of arms, the system operable to deliver microcurrents via skin tissue and muscle, including tendons, and/or to provide kinesiological sculpting, according to one illustrated implementation.

### DETAILED DESCRIPTION

In the following description, certain specific details are set forth in order to provide a thorough understanding of various disclosed implementations. However, one skilled in the relevant art will recognize that implementations may be practiced without one or more of these specific details, or with other methods, components, materials, etc. In other instances, well-known structures associated with power supplies, electrodes, circuitry and user interfaces have not been shown or described in detail to avoid unnecessarily obscuring descriptions of the implementations.

Unless the context requires otherwise, throughout the specification and claims that follow, the word "comprising" is synonymous with "including," and is inclusive or open-ended (*i.e.,* does not exclude additional, unrecited elements or method acts).

Reference throughout this specification to "one implementation" or "an implementation" means that a particular feature, structure or characteristic described in connection with the implementation is included in at least one implementation. Thus, the appearances of the phrases "in one implementation" or "in an implementation" in various places throughout this specification are not necessarily all referring to the same implementation. Furthermore, the particular features, structures, or characteristics may be combined in any suitable manner in one or more implementations.

As used in this specification and the appended claims, the singular forms "a," "an," and "the" include plural referents unless the context clearly dictates otherwise. It should also be noted that the term "or" is generally employed in its sense including "and/or" unless the context clearly dictates otherwise.

As used in this specification and the appended claims, the terms "muscle" or "muscles" is expansive and includes tendons as well as tissue that is strictly defined as muscles.

The headings and Abstract of the Disclosure provided herein are for convenience only and do not interpret the scope or meaning of the implementations.

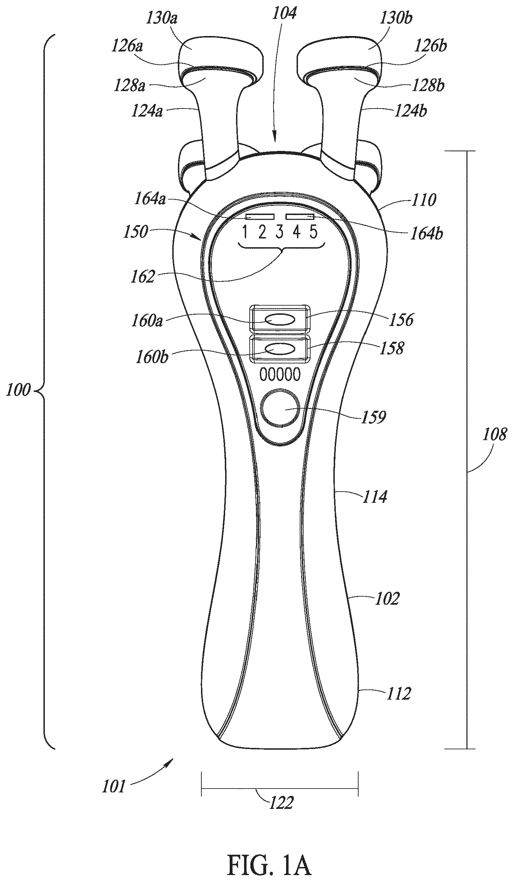

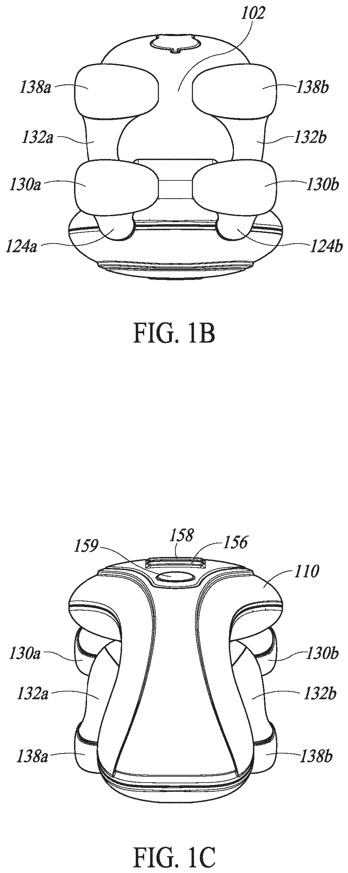

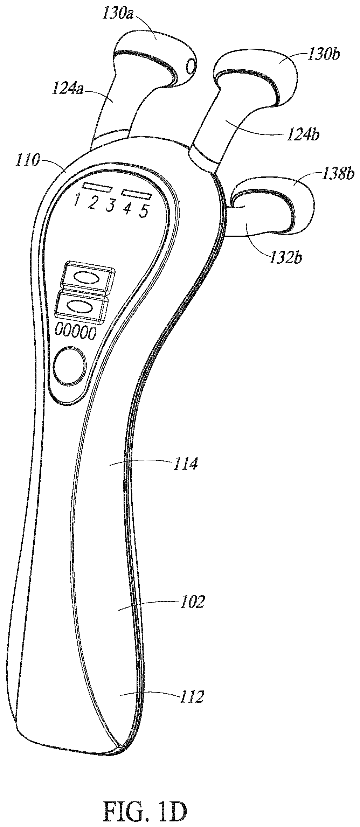

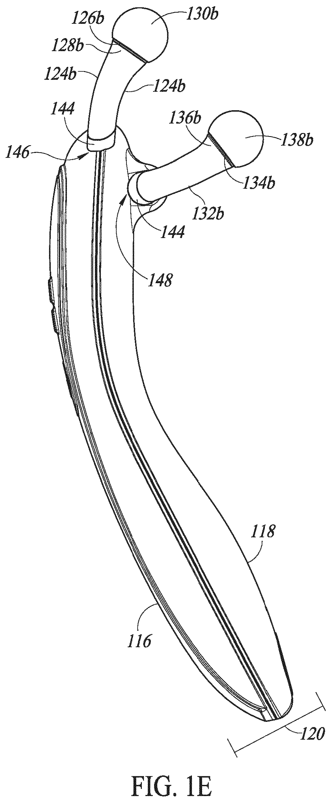

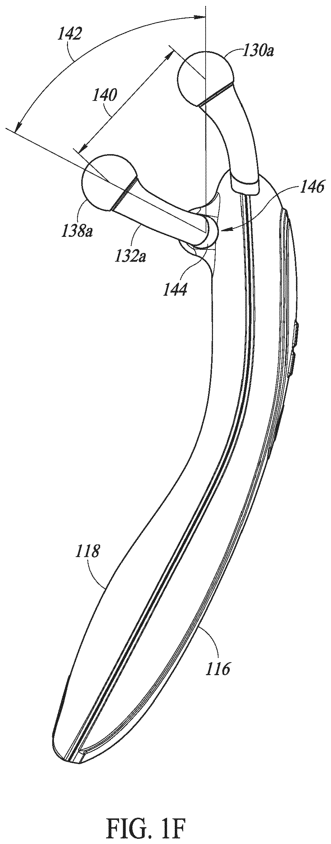

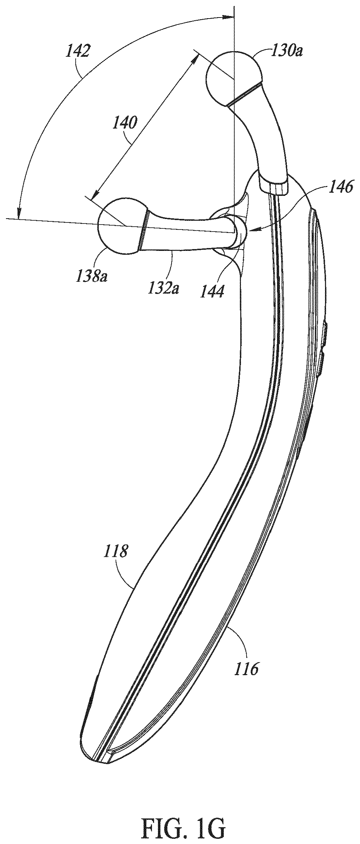

Figures 1A, 1B, 1C, 1D, 1E, 1F and 1G show a system 101 in the form of a handheld device 100 that is operable to delivery microcurrents (*e.g.,* signals with amperage at or below around 400 µA) via bodily tissue (*e.g.,* skin tissue, muscle including tendons) and/or which is also able to provide kinesiological sculpting via a stretching or tensioning and/or a squeezing or pinching of bodily tissue (*e.g.,* skin, underlying muscles including tendons), according to at least one illustrated implementation.

The handheld device 100 comprises a body 102 having a first end 104 and a second end 106, the second end 106 spaced from the first end 104 along a length 108 of the body 102. The body 102 can have a head 110 at the first end 104, a tail 112 at the second end 106, and handle portion 114 intermediate the head 110 and tail 112. The handle portion 114 has dimensions sized to be grasped in one hand of a user having average sized hands, hence the handheld device 100 is referred to as being handheld. The body 102 can have an upper surface 116 and a lower surface 118, the lower surface 118 generally opposed from the upper surface 116 across a thickness 120 of the body 102. The body 102 can have a somewhat arcuate profile along the length 108 thereof which can facilitate holding and positioning of the head 110 relative to bodily tissue for cosmetic treatment. The body 102 can have a width 122, that is generally laterally transverse to the length 108. The width 122 can vary in dimension as the length 108 is transversed. Likewise, the thickness 120 can vary in dimension as the length 108 is transversed.

The handheld device 100 includes a first pair of arms 124a, 124b that extend outwardly from the head 110 at the first end 104 of the body 102. The arms 124a, 124b of the first pair of arms 124a, 124b are spaced laterally apart from one another with respect to the width 122 of the body 102. Each of the arms 124a, 124b of the first pair of arms 124a, 124b has a respective terminus 126a, 126b at a distal end 128a, 128b of the arm 124a, 124b. Each of the arms 124a, 124b of the first pair of arms 124a, 124b has or bears or otherwise carries a respective electrode 130a, 130b positioned at least proximate the respective terminus 126a, 126b of the arm 124a, 124b. The electrodes 130a, 130b are preferably fixed to the respective arm 124a, 124b. In some implementations, the electrodes 130a, 130b can be replaceably removable from the respective arms 124a, 124b.

The handheld device 100 includes a second pair of arms 132a, 132b that extend outwardly from the head 110 at the first end 104 of the body 102. The arms 132a, 132b of the second pair of arms 132a, 132b are spaced laterally apart from one another with respect to the width 122 of the body 102. Each of the arms 132a, 132b of the second pair of arms 132a, 132b has a respective terminus 134a, 134b at a distal end 136a, 136b of the arm 132a, 132b. Each of the arms 132a, 132b of the second pair of arms 132a, 132b has or bears or otherwise carries a respective electrode 138a, 138b positioned at least proximate the respective terminus 134a, 134b of the arm 132a, 132b. The electrodes 138a, 138b are preferably fixed to the respective arm 132a, 132b to move therewith. In some implementations, the electrodes 138a, 138b can be replaceably removable from the respective arms 132a, 132b.

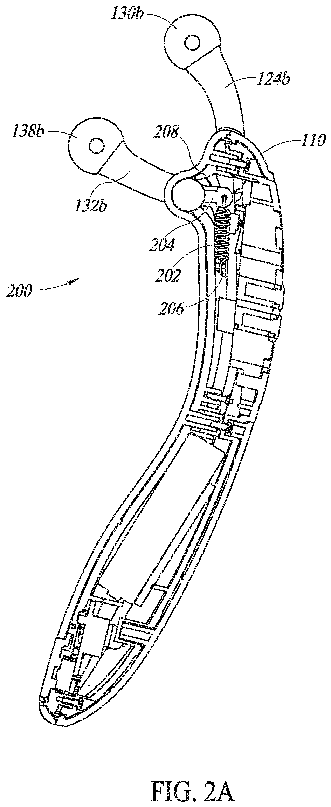

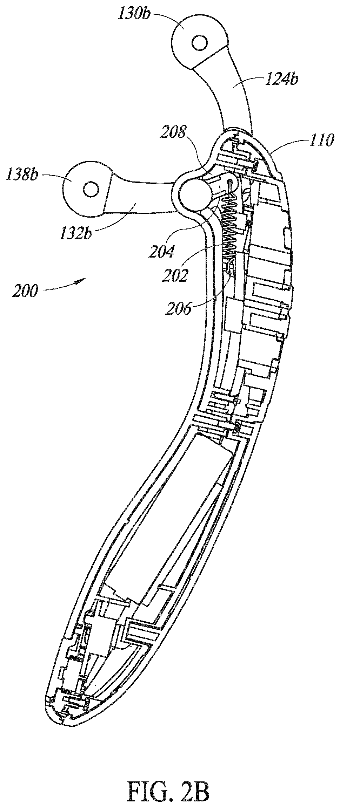

The arms 132a, 132b of the second pair of arms 132a, 132b are movable with respect to the arms 124a, 124b of the first pair of arms 124a, 124b. For example, the arms 132a, 132b of the second pair of arms 132a, 132b are movable with respect to the arms 124a, 124b of the first pair of arms 124a, 124b to vary a spacing 140 (best seen in Figures 1F and 1G and 2A and 2B) between the electrodes 138a, 138b of, borne by or otherwise carried by, the arms 132a, 132b of the second pair of arms 132a, 132b and the electrodes 130a, 130b of, borne by or otherwise carried by, the arms 124a, 124b of the first pair of arms 124a, 124b. Also for example, the arms 132a, 132b of the second pair of arms 132a, 132b are pivotable with respect to the arms 124a, 124b of the first pair of arms 124a, 124b to vary an angle 142 (best seen in Figures 1F and 1G and 2A and 2B) between the electrodes 138a, 138b of, borne by or otherwise carried by, the second pair of arms 132a, 132b and the electrodes 130a, 130b of, borne by or otherwise carried by the arms 124a, 124b of the first pair of arms 124a, 124b.

In at least one implementation, one pair of arms (*e.g.,* the first pair of arms 124a, 124b) are fixed or otherwise not moveable with respect to the body 102, while the other pair of arms (*e.g.,* the second pair of arms 132a, 132b) are movably with respect to the body 102, for example pivotable with respect thereto. In other implementations, both pairs of arms 124a, 124b; 132a, 132b can be moveable with respect to the body 102, and hence with respect to one another. In at least some implementations, the arms 124a, 124b that are fixed or do not pivot can each have a length of around 28.4 plus or minus 0.5 millimeters from base to geometric center of electrode 130a, 130b. In at least some implementations, the arms 132a, 132b that are moveable or pivotable can each have a length of around 27.7 plus or minus 0.5 millimeters from base to geometric center of electrode 138a, 138b.

In at least some implementations, each of the electrodes 130a, 130b, 138a, 138b has a generally frustro-conical shape, for instance with a profile of a truncated paraboloid or otherwise bullet shaped, for instance having an axis of symmetry or revolution that extends predominately laterally with respect to the length 108 of the body 102. The axis of symmetry or revolution can optionally angle slightly in a direction from a front to a rear of the handheld device 100 such the electrodes 130a, 130b, 138a, 138b toe in or angle in slightly toward a centerline of the handheld device 100. In at least some implementations, each of the bullet-shaped electrodes 130a, 130b, 138a, 138b has a width (*i.e.,* measured laterally) of around 20.76 millimeters plus or minus 0.5 millimeters. In at least some implementations, each of the bullet-shaped electrodes 130a, 130b, 138a, 138b has an outer portion (facing laterally outward with respect to the body 102) with a diameter of around 13.85 millimeters plus or minus 0.5 millimeters. For each pair of electrodes 130a, 130b; 138a, 138b, a lateral separation (*i.e.,* measured from inside edge to inside edge) between the electrodes of the pairs of electrode 130a, 130b; 138a, 138b can, for example be around 8.47 plus or minus 1.0 millimeters.

In at least some implementations, each of the electrodes 130a, 130b, 138a, 138b has a respective geometric center and a distance between the geometric centers of the electrodes of the second pair of electrodes 138a, 138b and corresponding ones of the electrodes of the first pair of electrodes 130a, 130b is approximately 34.8 millimeters plus or minus 0.5 millimeters in un-rotated orientation and is approximately 45.0 millimeters in the fully rotated orientation plus or minus 0.5 millimeters. In at least some implementations, each of the electrodes 130a, 130b, 138a, 138b has a respective geometric center and an angle between the electrodes of the second pair of electrodes 138a, 138b and corresponding ones of the electrodes of the first pair of electrodes 130a, 130b is approximately 63.4 degrees plus or minus 0.5 degrees in un-rotated orientation (Figure 1F, Figure 2A) and is approximately 88.5 degrees plus or minus 0.5 degrees in the fully rotated orientation (Figure 1G, Figure 2B).

The electrodes 130a, 130b, 138a, 138b are electrically conductive. For example, the electrodes 130a, 130b, 138a, 138b can advantageously comprise a stainless steel (*e.g.,* 420W) that is nickel free or has a low nickel content relative to other stainless steels (*e.g.,* austenitic stainless steels). In at least some implementations, the electrodes 130a, 130b, 138a, 138b of at least one pair of arms 124a, 124b; 132a, 132b, and preferably of both pairs of arms 124a, 124b, 132a, 132b, each advantageously have a rough exposed surface. The electrodes 130a, 130b, 138a, 138b can, for example, have an exterior surface that has been sandblasted with an abrasive to create the rough exposed surface. The surface roughness of the electrodes 130a, 130b, 138a, 138b can vary from electrode to electrode and/or vary across portions of a given electrode 130a, 130b, 138a, 138b. Some sample measurements of surface roughness can include a roughness depth of from about 0.29 microns to about 1.15 microns, inclusive.

As best illustrated in Figure 1A, the handheld device 100 can optionally include a user interface 150 accessible from an exterior 152 of the body 102. The user interface 150 can, for example, be carried on or in the upper surface 116 of the body 102. The user interface 150 can include a number of user selectable controls (*e.g.,* buttons, keys, switches) which are engageable to operate the handheld device 100. For example, the user interface 150 can include a power button 154 to, for example, toggle the handheld device 100 between an ON or powered state and an OFF or unpowered state. Also for example, the user interface 150 can include a first mode button (LIFT) 156 to, for example, cause the handheld device 100 to enter a first operational mode in which facial sculpting movements are performed using the handheld device 100 to physically lengthen and shorten muscles as a mechanism of action (kinesiology). Successive activations of the first mode button (LIFT) 156 can successively toggle through a set of levels of microcurrent amplitude or magnitude, to control an amplitude or magnitude of microcurrent signals delivered via the electrodes 130a, 130b, 138a, 138b while operating in the first mode. Also for example, the user interface 150 can include a second mode button (SKIN) 158 to, for example, cause the handheld device 100 to enter a second operational mode in which the device stimulate the mitochondria and achieves circulatory benefits. Successive activations of the first mode button (SKIN) 158 can successively toggle through a set of levels of amplitude or magnitude of microcurrent signals, to control an amplitude or magnitude of microcurrent delivered via the electrodes 130a, 130b, 138a, 138b while operating in the second mode.

The user interface 150 can include a number output devices or components (*e.g.,* lights, LEDs, speakers) to, for example, provide visual and/or aural indications to the user. For example, the user interface 150 can include a pair of operating mode indicators 160a, 160b that indicate an operating mode in which the handheld device 100 is currently operating. The operating mode indicators 160a, 160b can be incorporated into respective ones of the first and second mode buttons 156, 158. Also for example, the user interface 150 can include a charge level indicator 162 that indicates a charge condition of a power source (*e.g.,* secondary battery) of the handheld device 100, for instance on a scale of 1 to 5. Also for example, the user interface 150 can include a pair of continuity indicators 164a, 164b that indicate a presence or absence of continuity between the electrodes 130a, 130b, 138a, 138b when in contact with skin and in an ON or powered operational state. Such advantageously provides the user with a clear indication that the handheld device 100 is correctly applied to the skin and operable to deliver the microcurrent signals via the two pairs of electrodes 130a, 130b, 138a, 138b.

Figures 2A illustrates the handheld device 100 of Figure 1A showing the second pair of arms 132a, 132b in an un-rotated orientation or configuration relative to the first pair of arms 124a, 124b. Figure 2B illustrates the device of Figure 1A showing the second pair of arms 132a, 132b in a fully rotated orientation or configuration relative to the first pair of arms 124a, 124b.

As best illustrated in Figures 2A and 2B, the handheld device 100 further comprises at least one bias mechanism 200 housed by the body 102. The at least one bias mechanism 200 is coupled to bias the respective terminuses 134a, 134b of the arms 132a, 132b of the second pair of arms 132a, 132b toward the respective terminuses 126a, 126b of the arms 124a, 124b of the first pair of arms 124a, 124b. In at least some implementations, at least one bias mechanism 200 comprises a respective bias mechanism 200 for each of the arms 132a, 132b of the second pair of arms 132a, 132b. Alternatively, a single bias mechanism 200 can be employed to bias both arms 132a, 132b of the second pair of arms 132a, 132b.

Each bias mechanism 200 can respectively include at least one spring 202 (e.g., coil spring, leaf spring, tension spring) and optionally a lever 204, the spring 202 coupled between the respective lever 204 and an anchor feature 206 of the body 102. The at least one bias mechanism 200 biases the respective terminuses 134a, 134b and electrodes 138a, 138b of the arms 132a, 132b of the second pair of arms 132a, 132b toward an un-rotated orientation or configuration (Figures 1F, 2A) in which the respective terminuses 134a, 134b and electrodes 138a, 138b of the arms 132a, 132b of the second pair of arms 132a, 132b are at a smallest permitted distance with respect to the respective terminuses 126a, 126b and electrodes 130a, 130b of the arms 124a, 124b of the first pair of arms 124a, 124b. In at least some implementations, the spring 202 (*e.g.,* tension spring) can have a proportional constant or K value of, for example, around 1.881.95kgf/mm to around 1.95kgf/mm. Such advantageously applies a tension to the bodily tissue (*e.g.,* skin tissue, muscles underlying the skin tissue, including tendons) during use when the second pair of electrodes 138a, 138b are displaced from a default un-rotated position or orientation, to stretch and/or squeeze the bodily tissue to induce a kinesiological effect. It is noted that in at least some instances during use, the second pair of arms 132a, 132b may be partially rotated between the un-rotated orientation or configuration (Figures 1F, 2A) and the fully rotated orientation or configuration (Figures 1G, 2B). In use, as the second pair of electrodes 138a, 138b engage (*e.g.,* grab) the muscle, the spring 202 allows for the second pair of electrodes 138a, 138b to stay in contact with the tissue, while the body 102 of the handheld device 100 upward. Once the extension (*e.g.,* full extension) of the second pair of arms 132a, 132b is reached, the electrodes 130a, 130b of the first pair of arms 124a, 124b are gently put in contact with the tissue (*e.g.,* face). Because the electrodes 138a, 138b of the second pair of arms 132a, 132b is under tension, it continuously pushes the muscle upward with a specified force to facilitate kinesiological sculpting.

As best illustrated in Figures 2A and 2B, the device can further include one or more stops 208 that limits travel of the second pair of arms 132a, 132b when moving away from un-rotated orientation or configuration (Figure 1F) to set a fully rotated orientation or configuration (Figure 1G) in which the respective terminuses 134a, 134b and electrodes 138a, 138b of the arms 132a, 132b of the second pair of arms 132a, 132b are at a largest permitted distance and/or largest permitted angle with respect to the respective terminuses 126a, 126b and electrodes 130a, 130b of the arms 124a, 124b of the first pair of arms 124a, 124b.

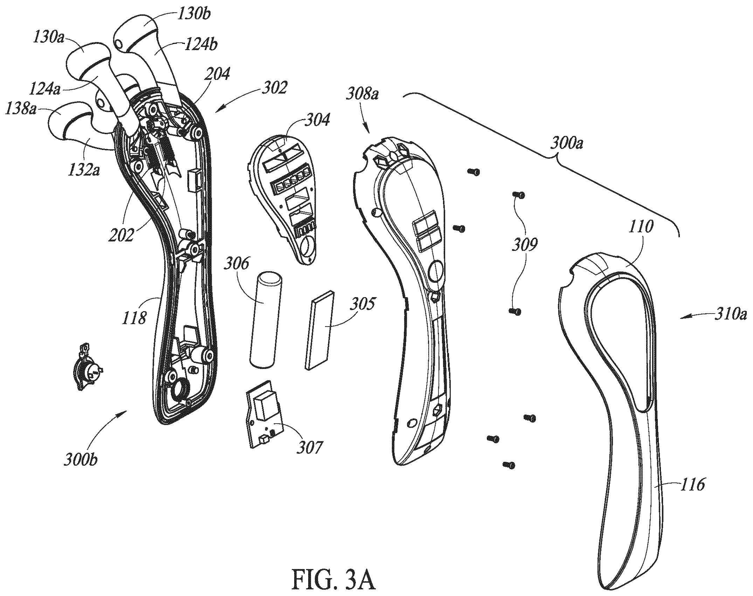

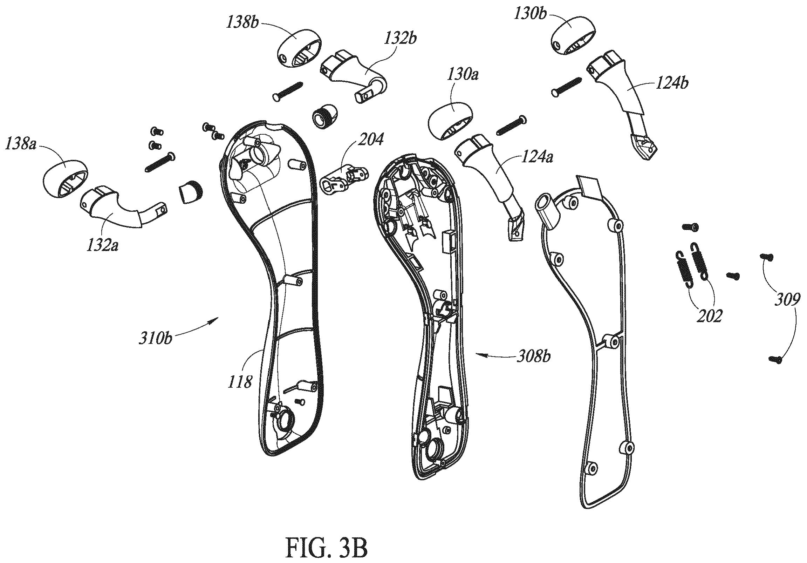

Figure 3A illustrates the device of Figure 1A in a first exploded view better illustrating an upper shell 300a. Figure 3B illustrates the device of Figure 1A in a second exploded view better illustrating a lower shell 300b.

The body 102 can be formed as a shell, for example a multi-part shell, for example comprising an upper shell 300a, a lower shell 300b, and an optional intermediate shell 300c, with an interior 302 to house circuitry 304, a power source 306, resilient cushion 305 to hold the power source 306 in a snug position, recharging circuit 307, etc. In some implementations, the upper shell 300a is coupled to the lower shell 300b via one or more fasteners 309 (only one called out, *e.g.,* screws, bolts) with the intermediate shell 300c positioned therebetween. In other implementations, the intermediate shell 300c can be omitted. The body 102 can include a portion that provides an electrically insulative enclosure protecting a user from potential contact with various circuit components and/or providing environmental protection for the circuit components (*e.g.,* printed circuit board (PCB), power source (*e.g.,* chemical battery cells, fuel cells, super- or ultra-capacitors), wiring, lights, speakers, haptic engine) and/or mechanical structures. For example, the body 102 can include an inner shell 308a, 308b, 308c formed from an electrically insulative material, for instance an injection molded plastic (*e.g.,* ABS plastic) shell. The body 102can optionally include an outer shell 310a, 310b, for example a metal shell that overlies respective ones of the inner shell 308a, 308b (*e.g.,* internal plastic shell). Such can advantageously increase wear resistance and/or provide some mass to make it easier to physically manipulate the handheld device 100 with controlled movements. Thus, in at least some implementations the upper shell 300a is comprised of outer shell 310a and inner shell 308a while the lower shell 300b is comprised of outer shell 310b and inner shell 308b. The handheld device 100 can include a number of seals (*e.g.,* gaskets, O-rings) 144 for example at each location 146 at which the first pair of arms 124a, 124b and/or the second pair of arms 132a, 132b joins or enters the body 102 to enhance environmental protection. There is a joint 148 located at least at the locations at which the second pair of arms 132a, 132b joins or enters the body 102, allowing the second pair of arms 132a, 132b to move (*e.g.,* pivot, rotate) relative to the body 102 and relative to the first pair of arms 124a, 124b.

The handheld device 100 optionally includes circuitry 304 that is communicatively coupled to the electrodes 130a, 130b of the first pair of arms 124a, 124b and the electrodes 138a, 138b of the second pair of arms 132a, 132b. The circuitry 304 is operable delivery at least one microcurrent signal via the electrodes 130a, 130b; 138a, 138b borne by the first and the second pairs of arms 124a, 124b; 132a, 132b. The circuitry 304 can be housed by the body 102. As previously noted a portion of the body 102 can provide an electrically insulative enclosure protecting a user from potential contact with various circuit components and/or providing environmental protection for the circuitry 304, for example an inner shell 308a, 308b, 308c formed from an electrically insulative material for instance an injection molded plastic (ABS plastic) shell.

The circuitry 304 can, for example, be operable to deliver: i) a first microcurrent signal between a first one of the electrodes of the first pair of electrodes 130a, 130b and a first one of the electrodes of the second pair of electrodes 138a, 138b, ii) a second microcurrent signal between a second one of the electrodes of the first pair of electrodes 130a, 130b and a second one of the electrodes of the second pair of electrodes 138a, 138b, iii) a third microcurrent signal between the first one of the electrodes of the first pair of electrodes 130a, 130b and the second one of the electrodes of the second pair of electrodes 138a, 138b, and iv) a fourth microcurrent signal between the second one of the electrodes of the first pair of electrodes 130a, 130b and the second one of the electrodes of the second pair of electrodes 138a, 138b. In at least some implementations or configurations, the circuitry 304 is operable to deliver the first microcurrent signal, the second microcurrent signal, the third microcurrent signal and the fourth microcurrent signal concurrently with one another. The circuitry 304 can, for example, be operable to adjust an amplitude or magnitude of the microcurrent, for instance based in input received via the user interface 150 (Figure 1A).

In at least some implementations or configurations, the circuitry 304 is operable to deliver the first microcurrent signal with a first set of frequencies, and deliver at least one of the second microcurrent signal, the third microcurrent signal or the fourth microcurrent signal with a second set of frequencies, the second set of frequencies different than the first set of frequencies.

In at least some implementations or configurations, the circuitry 304 is operable to deliver the first microcurrent signal with a first set of frequencies, deliver the second microcurrent signal with a second set of frequencies, deliver the third microcurrent signal with a third set of frequencies, and deliver the fourth microcurrent signal with a fourth set of frequencies, the second set of frequencies different than the first set of frequencies, the third set of frequencies different than the first and the second sets of frequencies; and the fourth set of frequencies different than the first, the second and the third set of frequencies.

The circuitry 304 can include a frequency generator, pulse generator, pulse envelope generator and a controller to provide controlled current in each channel from about 20 µA to about 400 µA at up to 300 Hz. The circuitry 304 is electrically coupled to a power supply or power source to receive electrical power therefrom. Two or more frequencies are used to provide interferential wave forms.

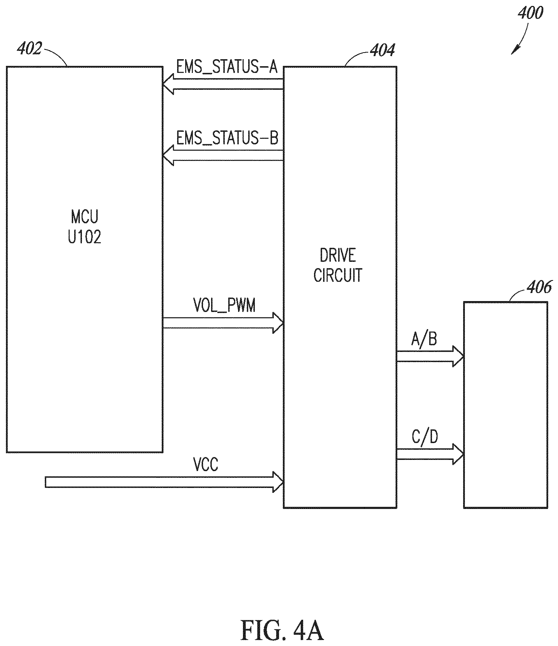

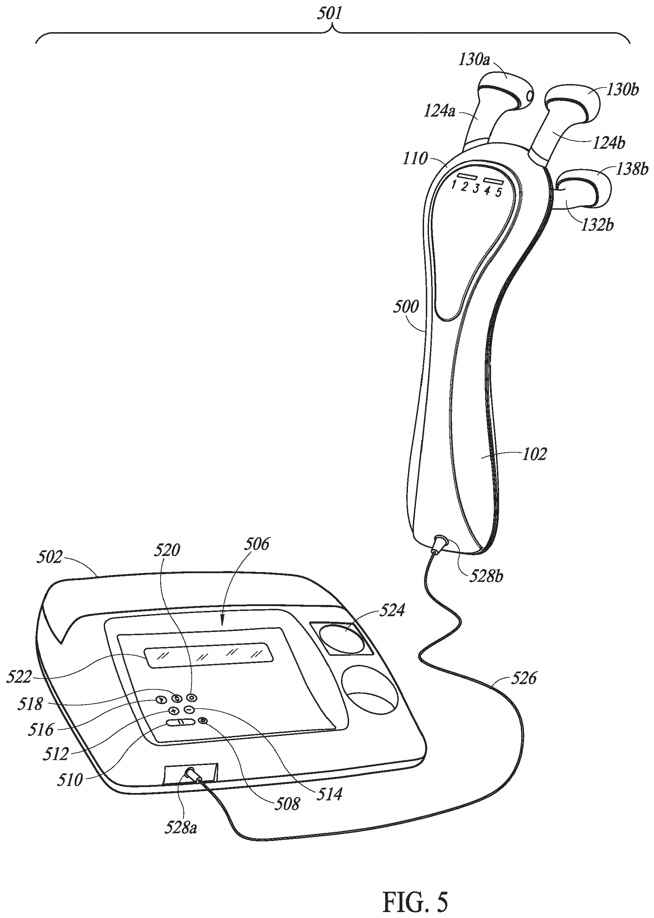

Figure 4A shows circuitry 400 of the system 101 (Figures 1A-1G), 501 (Figure 5), according to at least one illustrated implementation. The circuitry 400 can form a portion of the circuitry 304 (Figure 3A).

The circuitry 400 includes a microprocessor control unit ("MCU") 402. The MCU 402 receives inputs from various switches, *e.g.,* via keys, buttons 159, 156, 158 or other user input devices of the user interface 150 (Figures 1A) and provides signals to various indicators (*e.g.,* LEDs, LCD, speakers) 160a, 160b, 162, 164a, 164b, 166, or other output devices of the user interface 150 (Figures 1A). The MCU 402 receives inputs from various sensors (*e.g.,* continuity sensors, current sensors, voltage sensors), as well as receiving power from a power source 306 (Figure 3A).

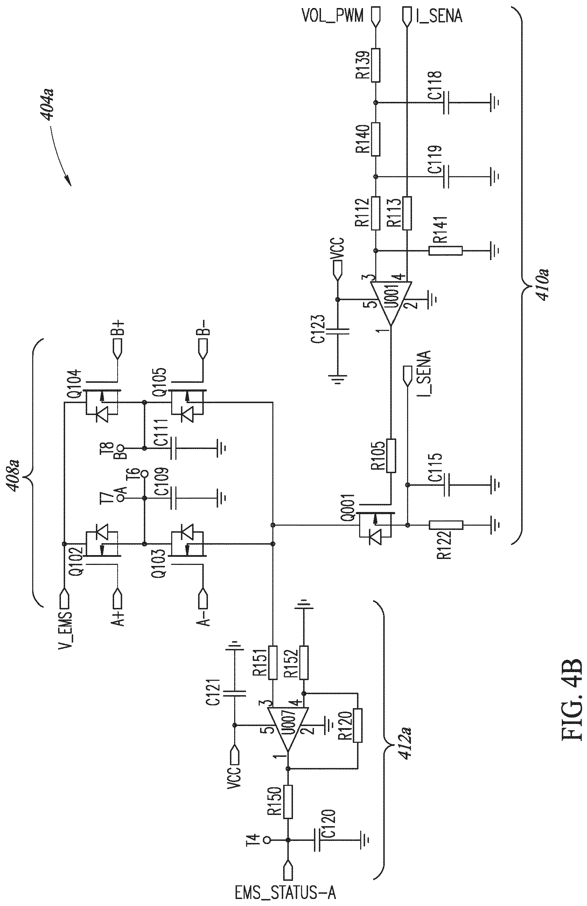

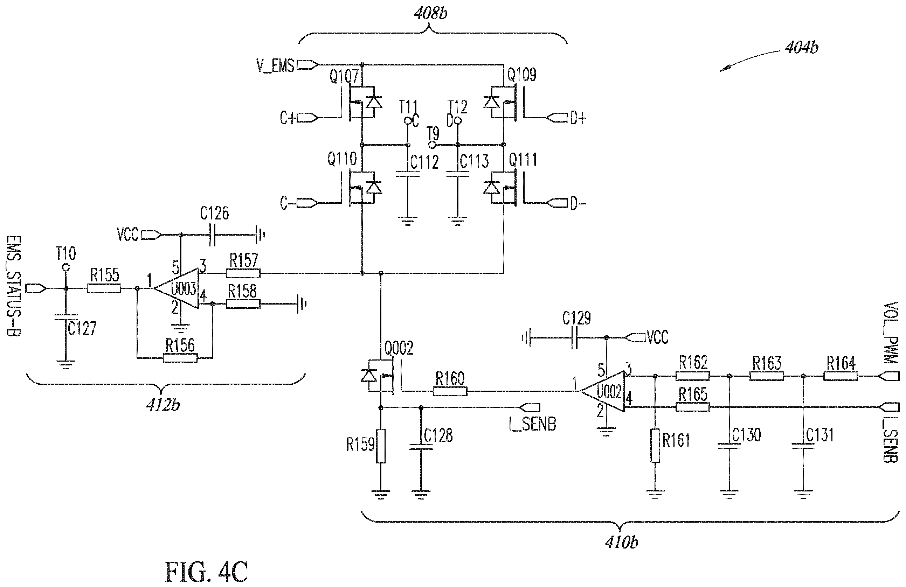

The circuitry 400 includes a drive circuit 404, which as explained below can have two distinct portions. In use, the MCU 402 provides a pulse width modulated signal (VOL PWM) to the drive circuit 404 which is represented in more detail in Figures 4B and 4C, respectively. The drive circuit 404 receives power VCC from a power supply, and provides microcurrent signals (A/B; C/D) to electrodes 406 (shown collectively for ease of illustration). The drive circuit 404 can also monitor the microcurrent signals A/B, providing status signals (EMS_STATUS-A; EMS_STATUS-B) to the MCU 402.

Figure 4B shows in detail a first portion 404a of the drive circuit 404 of the system 101 (Figures 1A-1G), 501 (Figure 5), according to at least one illustrated implementation. The first portion 404a of the drive circuit 404 can form a portion of the circuitry 304 (Figure 3A).

The first portion 404a of the drive circuit 404 includes a bridge 408a formed by four transistors Q102, Q103, Q104, Q105, which is driven by gate drive signals A+, A-, B+, B- to provide microcurrent signals via nodes or terminals T7, T8, which are coupled to ground via capacitors C102, C111. Extra nodes or terminals (*e.g.,* T6) can optionally be included to provide additional channels of microcurrent signals. The gate drive signals A+, A-, B+, B- provide frequency modulation of the microcurrent signals.

The first portion 404a of the drive circuit 404 includes a magnitude adjustment circuit 410a comprised of a transistor Q001 which is coupled to ground via a resistor R122 and a capacitor C115, which provides a sensed current output I_SENA. The transistor Q001 is driven via gate drive signals generated via an output terminal 1 of a voltage comparator U001 which are applied to a gate of the transistor Q001 via a resistor R105. The voltage comparator U001 compares a pulse width modulated (PWM) signal VOL_PWM supplied comparator U001 compares a pulse width modulated (PWM) signal VOL_PWM supplied to one input terminal 3 of the comparator U001 with the sensed current I_SENSA supplied to another input terminal 4 of comparator U001. The comparator U001 can be implemented via an operational amplifier, with voltage sampling value I_SENS input to an inverting terminal, to be compared with a standard VOL_PWM, and the output terminal coupled to control the gate of transistor Q001, to achieve accurate control of the current level of the microcurrent signal. A network of resistors R139, R140, R141, R112, R113 and capacitors C118, C119 couples the PWM signal VOL_PWM and sensed current I_SENSA to the comparator U001.

The first portion 404a of the drive circuit 404 includes a status monitoring circuit 412a that provides the status signal (EMS_STATUS-A) to the MCU 402 (Figure 4A). The status monitoring circuit 412a employs a comparator U007 to monitor the microcurrent output of the principal channel (A).

Figure 4C shows in detail a second portion 404b of the drive circuit 404 of the system 101 (Figures 1A-1G), 501 (Figure 5), according to at least one illustrated implementation. The second portion 404b of the drive circuit 404 can form a portion of the circuitry 304 (Figure 3A).

The second portion 404b of the drive circuit 404 includes a bridge 408b formed by four transistors Q107, Q109, Q110, Q111, which is driven by gate drive signals C+, C-, D+, D- to provide microcurrent signals via nodes or terminals T11, T12, which are coupled to ground via capacitors C112, C113. Extra nodes or terminals (*e.g.,* T9) can optionally be included to provide additional channels of microcurrent signals. The gate drive signals C+, C-, D+, D-provide frequency modulation of the microcurrent signals.

The second portion 404b of the drive circuit 404 includes a magnitude adjustment circuit 410b comprised of a transistor Q002 which is coupled to ground via a resistor R159 and a capacitor C128, which provides a sensed current output I_SENB. The transistor Q002 is driven via gate drive signals generated via an output terminal 1 of a voltage comparator U002 which are applied to a gate of the transistor Q002 via a resistor R160. The voltage comparator U002 compares a pulse width modulated (PWM) signal VOL_PWM supplied to one input terminal 3 of the comparator U002 with the sensed current I_SENSB supplied to another input terminal 4 of comparator U002. The comparator U002 can be implemented via an operational amplifier, with voltage sampling value I_SENS input to an inverting terminal, to be compared with a standard VOL_PWM, and the output terminal coupled to control the gate of transistor Q002, to achieve accurate control of the current level of the microcurrent signal. A network of resistors R161, R162, R163, R164, R165 and capacitors C130, C131 couples the PWM signal VOL_PWM and sensed current I_SENSB to the comparator U002.

The second portion 404b of the drive circuit 404 includes a status monitoring circuit 412b that provides the status signal (EMS_STATUS-B) to the MCU 402 (Figure 4A). The status monitoring circuit 412b employs a comparator U003 to monitor the microcurrent output of the principal channel (B).

The circuitry 400 provides low frequency and amplitude wave forms to aid fluid flow in a subject's bodily tissue to enhance ATP production and hence repair of tissue. The device 100 provides pulsed energy envelopes of micro current with a mandatory pause between pulses. The wave forms are preferably modulated by a fifty percent duty cycle square wave.

The circuitry 400 can, for example, include a microprocessor control unit ("MCU") that controls analog output circuitry and instrumentation circuitry, a power supply, a user interface (*e.g.*, a control panel with a display or visual indicators (*e.g.,* LEDs) and buttons or keys, and optionally an audio speaker. The analog output circuitry includes a number of output leads 11 to transfer microcurrent signals to the electrodes. The MCU can include a microprocessor, volatile memory for instance random access memory ("RAM"), non-volatile memory for instance read-only memory ("ROM") or FLASH memory, analog to digital conversion ("ADC"), digital to analog conversion ("DAC"), computation, time-keeping and communications components.

The power supply can take the form of a switching power supply that can generate plus or minus 32 volts for a high voltage output operational amplifies (not shown), plus or minus 9 volts for an instrumentation operational amplifier, plus 5 volts for the microprocessor. The power can be supplied by batteries having a nominal working voltage of 12 volts or by any other source. The power is turned ON by a button and preferably turns OFF automatically after about six minutes, if no wave forms are being generated. The microprocessor also can control the ON-off status of the power supply.

The analog output circuitry is used to supply the current across a channel under control of the MCU. The circuitry can be similar to that illustrated and described in U.S. Patent 5,817,138 or European Patent 1009478. The output stages can comprise operational amplifies (op amps) in a voltage controlled constant current configuration with a maximum current capability of 180 µA at 30 volts.

The MCU controls the voltage with a DAC which connects to the op amp to set the output current of the op amps. The DAC allows programmable ramp up of the current. An in-line voltage multiplier controls the on/off status and polarity of the output stage. The output current flow is controlled with the op amp circuit only, after the voltages are set.

The user interface or control panel allows for selection or entry of desired parameters and pre-programmed treatment settings that are groups of parameters predetermined for particular treatments, and for displaying various treatment parameters such as time, current, voltage, etc. Convenient buttons are used for turning on the device and entering parameters and settings.

The microprocessor can produce wave forms with selected envelopes, modulating frequencies, and polarity. Each channel can be independently controlled. Each output channel is a separate operational op amp circuit, with signal wiring physically isolated from the other channels using, for example, 1 Meg ohms between channels.

The electrical output of the micro current device is a wave form, representing the current as measured across a 10k ohm resistor. The wave form is typically a complex wave form. In general, all wave forms consist of a selectable predetermined wave form envelope, which is then modulated with a 50% duty square wave, of the frequency selected.

In one implementation, a standard wave form (of two complete cycles) consists of an envelope of 2.0 second negative square wave with a 0.5 second pause and then a positive wave form for 2.0 seconds. For example, this wave form envelope can be modulated by a 2.2 Hz frequency 50% duty cycle signal. The modulating frequency of 2.2 and 1.3 Hz signal is referred to as the frequency of the output wave form is selectable, *i.e.,* other modulation frequencies can be selected. The wave shape envelope does not change due to frequency and is always fixed at 2.0 second square wave, with a 0.5 second pause, then another 2.0 second square wave with a 0.5 second pause, etc. As used, herein a pulse is one cycle, e.g., a wave shape envelope of negative polarity or a wave shape envelope of positive polarity.

The MCU 10 supplies the intelligence to run the system 101 (Figure 1A), 501 (Figure 5). In addition to control functions, the MCU takes inputs from the user interface and provides output to the user.

The MCU sets the H-Bridge circuits to provide a user selected amount of current. The current is controlled in a constant mode by the H-Bridge feedback circuits. The amplitude or magnitude of the current can, for example, be selected in five discrete steps - 40 µA, 80 µA, 100 µA, 160 µA and 180 µA by setting the control voltage to the H-Bridge op amp. This can be accomplished through an associated op amp feedback circuitry. Current cannot exceed the selected set value or a specified maximum value (*e.g.,* 180 µA) under any circumstances using the circuitry to ensure safety.

A waveform envelope is controlled by a MCU pulse width modulator "PWM" and the filter circuit feeding analog switches, which sets the maximum current output. This means that after a maximum current is selected (by the switches), it can be reduced as necessary to control the waveform by the microprocessor using its PWM output.

The MCU provides control of the H-Bridges through output circuitry for one A and one B channel. The device basically has four channels for output current signals according to of each the A and B channels illustrated. All channels having an "A" output are the same and all channels having a "B" output are the same.

A polarity of the output current is controlled in the H-Bridge by reversing the output transistor connections with the analog switches in the output circuit. Output frequency is controlled by cycling these both switches ON and OFF as the chosen frequency may require. Normally, one switch is ON and the other is OFF for current flow.

All H-Bridge circuits as commanded produce the same current as selected by the user, e.g., between 40 and 180 micro amperes.

All side A channels are driven with the same signal and all B channels are driven by a different same signal. Thus, side A outputs produce different output frequency characteristics from side B outputs. Each channel can be individually programmed, if desired, so that each channel has different output frequency characteristics. Typically, four channels (side A) have one output frequency characteristic and the other four channels (side B) have a second frequency characteristic.

Figure 5 shows a system 501 comprising a handheld device 500 and a console 502, the handheld device 500 communicatively coupled to the console 502, the system 501 operable to deliver microcurrents via skin tissue and muscle, including tendons, and/or to provide kinesiological sculpting, according to one illustrated implementation.

The console 502 can include all or some of the circuitry and/or electronics (*e.g.,* circuitry 304, Figure 3A; circuitry 400, Figures 4A-4C) that generate and supplies the microcurrent signals to electrodes 130a, 130b, 130c, 130d of a handheld device 500 for application to skin or other bodily tissue. Consequently, the handheld device 500 can omit all or some of the circuitry and/or electronics from previously described implementations, for instance where such circuitry and/or electronics are included or housed by the console 502. The handheld device 500 includes a body 102, head 110, and the arms 124a, 124b of the first pair of arms 124a, 124b and the arms 132a, 132b of the second pair of arms 132a, 132b, along with the electrodes 130a, 130b, 130c, 130d, where at least one pair of arms 124a, 124b; 132a, 132b is moveable (*e.g.,* pivotable) with respect to the other pair of arms 124a, 124b; 132a, 132b similar to that of the implementation of Figures 1A-1G, 2A, 2B, and 3A. In particular, at least one pair of arms 132a, 132b is moveable within a defined range of motion (*e.g.,* defined range of rotation or spacing) with respect to the other pair of arms 124a, 124b.

The console 502 can, for example, include a microprocessor control unit ("MCU") similar or identical to MCU 402 (Figure 4A). The console 502 can, for example, include, a drive circuit similar or identical to drive circuit 404 Figure 4A). The console 502 can, for example, include various switches (*e.g.,* keys, buttons, triggers) or other user input devices of a user interface 506, for example a power switch 508, a pause switch 510, a level or magnitude decrease switch 512, a level or magnitude increase switch 514, a mode selection switch 516, a program selection switch 518 and an option selection switch 520). The console 502 can, for example, include various indicators (*e.g.,* LEDs, LCD, speakers) or other output devices of the user interface 150, for example a display panel 522 and speaker 524. Alternatively, the handheld device 500 can carry one, more or all of the switches and/or indicators. The console 502 can, for example, include various sensors (*e.g.,* continuity sensors, current sensors, voltage sensors). Alternatively, the handheld device 500 can include one, more or all of the sensors (*e.g.,* continuity sensors, current sensors, voltage sensors).

The handheld device 500 can be communicatively coupled to the console 502 via one or more wires or cables 526. For example, the console 502 and/or the handheld device 500 can include one or more ports 528a, 528b (*e.g.,* wired ports, e.g., USB-C compliant ports or connectors) to which one or more wires or cables 526 are either detachable or permanently coupled. The wires or cables 526 can, for example, supply microcurrents from the console 502 to the electrodes 130a, 130b, 130c, 130d of the handheld device 500. The wires or cables 526 can, for example, optionally supply signals from the handheld device 500 to the console 502, for example signals indicative of continuity, current and, or voltage either to sensors housed by the console 502 or from sensors housed by the handheld device 500. Additionally or alternatively, radios, transmitters, receivers, and/or transceivers can communicatively couple handheld device 500 with the console 502. For instance, in some implementations the radios, transmitters, receivers, and/or transceivers can be employed to transmit sensed information between the handheld device 500 and the console 502.

Described herein are implementations and embodiments of a device that advantageously takes the form a handheld body with two pairs of electrodes mounted or carried by respective arms extending from the handheld body, where at least one pair of arms is movable (*e.g.,* pivotal, rotatable) with respect the handheld body and hence with respect to the other pair of arms, which is operable to deliver microcurrent signal via two separate channels and via interferential signals between the two separate channels, to facilitate kinesiology facial sculpting application.

It should be apparent that the above described method can include additional acts, omit some acts, and, or execute acts in a different order.

The foregoing detailed description has set forth various implementations of the devices and/or processes via the use of block diagrams, schematics, and examples. Insofar as such block diagrams, schematics, and examples contain one or more functions and/or operations, it will be understood by those skilled in the art that each function and/or operation within such block diagrams, flowcharts, or examples can be implemented, individually and/or collectively, by a wide range of hardware, software, firmware, or virtually any combination thereof. In one implementation, the present subject matter may be implemented via Application Specific Integrated Circuits (ASICs).

Those of skill in the art will recognize that many of the methods or algorithms set out herein may employ additional acts, may omit some acts, and/or may execute acts in a different order than specified.

In addition, those skilled in the art will appreciate that the mechanisms taught herein are capable of being distributed as a program product in a variety of forms, and that an illustrative implementation applies equally regardless of the particular type of signal bearing media used to actually carry out the distribution. Examples of signal bearing media include, but are not limited to, the following: recordable type media such as floppy disks, hard disk drives, CD ROMs, digital tape, and computer memory.

The various implementations described above can be combined to provide further implementations. To the extent that they are not inconsistent with the specific teachings and definitions herein, all of the U.S. patents, U.S. patent application publications, U.S. patent applications, foreign patents, foreign patent applications and non-patent publications referred to in this specification, including: U.S. Patent 5,817,138, International Patent Application Publication WO1998023326 A1; EP Patent EP1009478 B1, and U.S. patent application 18/148,140. Aspects of the implementations can be modified, if necessary, to employ systems, circuits and concepts of the various patents, applications and publications to provide yet further implementations.

## Claims

1. A system (101), comprising:
a body (102) having a first end (104) and a second end (106), the second end (106) spaced from the first end (104) along a length (108) of the body (102);
a first pair of arms (124b) (124a) that extend outwardly from the first end (104) of the body (102), the arms (124b) (124a) of the first pair of arms (124b) (124a) spaced laterally apart from one another with respect to a width (122) of the body (102), each of the arms (124b) (124a) of the first pair of arms (124b) (124a) having a respective terminus at a distal end (128b) (128a) of the arm and each of the arms (124b) (124a) of the first pair of arms (124b) (124a) having a respective electrode positioned at least proximate the respective terminus of the arm;
a second pair of arms (132b) (132a) that extend outwardly from the first end (104) of the body (102), the arms (132b) (132a) of the second pair of arms (132b) (132a) spaced laterally apart from one another with respect to a width (122) of the body (102), each of the arms (132b) (132a) of the second pair of arms (132b) (132a) having a respective terminus at a distal end (136b) (136a) of the arm and each of the arms (132b) (132a) of the second pair of arms (132b) (132a) having a respective electrode positioned at least proximate the respective terminus of the arm, wherein the arms (132b) (132a) of the second pair of arms (132b) (132a)are pivotable with respect to the arms (124b) (124a) of the first pair of arms (124b) (124a) to vary an angle (142) between the electrodes (138a) (130b) of the arms (132b) (132a) of the second pair of arms (132b) (132a) and the electrodes (130a) (130b) of the arms (124b) (124a) of the first pair of arms (124b) (124a);
at least a first pair of joints (148), the joints of the first pair of joints respectively pivotally couple respective ones of the arms (132b) (132a) of the second pair of arms (132b) (132a) to the body (102) at a first joint location (146) of the body (102) and a second joint location (146) of the body (102) respectively, the first joint location (146) and the second joint location (146) different from one another; and
at least one bias mechanism (200) coupled to bias the arms (132b) (132a) of the second pair of arms (132b) (132a) toward an un-rotated orientation in which an angle (142) between the arms (132b) (132a) of the second pair of arms (132b) (132a) and corresponding ones of the arms (124b) (124a) of the first pair of arms (124b) (124a) is a smallest angle (142) achievable therebetween.

2. The system (101) of claim 1 wherein at least one bias mechanism (200) comprises at least one spring (202).

3. The system (101) of claim 1 wherein at least one bias mechanism (200) applies a tension to bodily tissue when the electrodes (130a) (130b) (138a) (138b) of the first and the second pairs of arms (124b) (124a) (132b) (132a) are applied to skin tissue to achieve a kinesiological effect.

4. The system (101) of claim 1, further comprising:
a stop that limits rotation of the second pair of arms (132b) (132a) relative to the first pair of arms (124b) (124a) to a fully rotated orientation in which an angle (142) between the arms (132b) (132a) of the second pair of arms (132b) (132a) and corresponding arms (124b) (124a) of the first pair of arms (124b) (124a) is a largest angle (142) achievable.

5. The system (101) of claim 1 wherein each of the electrodes (130a) (130b) (138a) (138b) has a respective geometric center and an angle (142) between the electrodes (138a) (138b) of the second pair and corresponding ones of the electrodes (406) (138b) (130a) (130b) of the first pair is 63.4 degrees plus or minus 0.5 degrees in un-rotated orientation and is 88.5 degrees plus or minus 0.5 degrees in a fully rotated orientation.

6. The system (101) of claim 1 wherein the electrodes (130a) (130b) borne by the first pair of arms (124b) (124a) and the electrodes (138a) (138b) borne by the second pair of arms (132b) (132a) each have a rough exposed surface.

7. The system (101) of claim 1, further comprising:
a circuit (307), the circuit (307) communicatively coupled to the electrodes (130a) (130b) borne by the first pair of arms (124b) (124a) and the electrodes (138a) (138b) borne by the second pair of arms (132b) (132a), the circuit (307) operable to deliver a microcurrent via the electrodes (130a) (130b) (138a) (138b) borne by the first and the second pairs of arms (124b) (124a) (132b) (132a), wherein the circuit (307) is operable to deliver: i) a first microcurrent signal between a first one of the electrodes of the first electrode pair (130a) (130b) and a first one of the electrodes of the second electrode pair (138a) (138b), ii) a second microcurrent signal between a second one of the electrodes of the first electrode pair (130a) (130b) and a second one of the electrodes of the second electrode pair (138a) (138b), iii) a third microcurrent signal between the first one of the electrodes of the first electrode pair (130a) (130b) and the second one of the electrodes of the second electrode pair (138a) (138b), and iv) a fourth microcurrent signal between the second one of the electrodes of the first electrode pair (130a) (130b) and the second one of the electrodes of the second electrode pair (138a) (138b).

8. The system (101) of claim 7 wherein the circuit (307) is operable to deliver the first microcurrent signal, the second microcurrent signal, the third microcurrent signal and the fourth microcurrent signal concurrently with one another, and the first microcurrent signal has a first set of frequencies, the second microcurrent signal has a second set of frequencies, the third microcurrent signal has a third set of frequencies, and the fourth microcurrent signal has a fourth set of frequencies, the second set of frequencies different than the first set of frequencies, the third set of frequencies different than the first and the second sets of frequencies; and the fourth set of frequencies different than the first, the second and the third set of frequencies.

9. The system (101) of claim 7 wherein the body (102), the first pair of arms (124b) (124a) and the second pair of arms (132b) (132a) comprise a handheld device (500) (100) and the circuit (307) is housed by the body (102) of the handheld device (500) (100).

10. The system (101) of claim 7 wherein the body (102), the first pair of arms (124b) (124a) and the second pair of arms (132b) (132a) comprise a handheld device (500) (100), and further comprising:
a console (502) to which the body (102) is communicatively coupled, wherein the circuit (307) is housed by the console (502).

11. The system (101) of claim 1 wherein the body (102) is a handle body (102), having has dimensions sized to be grasped in one hand of a user having average sized hands, and a user interface (506) (150) accessible from an exterior (152) of the body (102).

12. The system (101) of claim 1 wherein the first pair of arms (124b) (124a) are fixedly coupled to the body (102) and do not pivot with respect to the body (102), and further comprising:
a pair of seals, each of the seals sealing a respective joint of the first pair of joints (148) at which a respective one of the arms (132b) (132a) of the second pair of arms (132b) (132a) is pivotally coupled to the body (102).

## Patentansprüche

1. System (101), umfassend:
einen Körper (102), der ein erstes Ende (104) und ein zweites Ende (106) aufweist, wobei das zweite Ende (106) vom ersten Ende (104) entlang einer Länge (108) des Körpers (102) beabstandet ist;
ein erstes Paar von Armen (124b) (124a), die sich vom ersten Ende (104) des Körpers (102) nach außen erstrecken, wobei die Arme (124b) (124a) des ersten Paars von Armen (124b) (124a) in Bezug auf eine Breite (122) des Körpers (102) seitlich voneinander beabstandet sind, wobei jeder der Arme (124b) (124a) des ersten Paars von Armen (124b) (124a) einen jeweiligen Endpunkt an einem distalen Ende (128b) (128a) des Arms aufweist und jeder der Arme (124b) (124a) des ersten Paars von Armen (124b) (124a) eine jeweilige Elektrode aufweist, die mindestens in der Nähe des jeweiligen Endpunkts des Arms positioniert ist;
ein zweites Paar von Armen (132b) (132a), die sich vom ersten Ende (104) des Körpers (102) nach außen erstrecken, wobei die Arme (132b) (132a) des zweiten Paars von Armen (132b) (132a) in Bezug auf eine Breite (122) des Körpers (102) seitlich voneinander beabstandet sind, wobei jeder der Arme (132b) (132a) des zweiten Paars von Armen (132b) (132a) einen jeweiligen Endpunkt an einem distalen Ende (136b) (136a) des Arms aufweist und jeder der Arme (132b) (132a) des zweiten Paars von Armen (132b) (132a) eine jeweilige Elektrode aufweist, die mindestens in der Nähe des jeweiligen Endpunkts des Arms positioniert ist, wobei die Arme (132b) (132a) des zweiten Paars von Armen (132b) (132a) in Bezug auf die Arme (124b) (124a) des ersten Paars von Armen (124b) (124a) schwenkbar sind, um einen Winkel (142) zwischen den Elektroden (138a) (130b) der Arme (132b) (132a) des zweiten Paars von Armen (132b) (132a) und den Elektroden (130a) (130b) der Arme (124b) (124a) des ersten Paars von Armen (124b) (124a) zu variieren;
mindestens ein erstes Paar von Gelenken (148), wobei die Gelenke des ersten Paars von Gelenken jeweils jeweilige der Arme (132b) (132a) des zweiten Paars von Armen (132b) (132a) zum Körper (102) an einer ersten Gelenkstelle (146) des Körpers (102) und an einer zweiten Gelenkstelle (146) des Körpers (102) schwenken, wobei sich jeweils die erste Gelenkstelle (146) von der zweiten Gelenkstelle (146) unterscheidet, und
mindestens einen Vorspannmechanismus (200), der zum Vorspannen der Arme (132b) (132a) des zweiten Paars von Armen (132b) (132a) zu einer ungedrehten Ausrichtung gekoppelt ist, bei der ein Winkel (142) zwischen den Armen (132b) (132a) des zweiten Paars von Armen (132b) (132a) und entsprechenden der Arme (124b) (124a) des ersten Paars von Armen (124b) (124a) ein kleinster Winkel (142) ist, der dazwischen erzielbar ist.

2. System (101) nach Anspruch 1, wobei mindestens ein Vorspannmechanismus (200) mindestens eine Feder (202) umfasst.

3. System (101) nach Anspruch 1, wobei mindestens ein Vorspannmechanismus (200) eine Spannung an Körpergewebe anlegt, wenn die Elektroden (130a) (130b) (138a) (138b) des ersten und des zweiten Paars von Armen (124b) (124a) (132b) (132a) an Hautgewebe angelegt werden, um eine kinesiologische Wirkung zu erzielen.

4. System (101) nach Anspruch 1, weiter umfassend:
einen Anschlag, der eine Drehung des zweiten Paars von Armen (132b) (132a) relativ zum ersten Paar von Armen (124b) (124a) auf eine vollständig gedrehte Ausrichtung begrenzt, bei der ein Winkel (142) zwischen den Armen (132b) (132a) des zweiten Paars von Armen (132b) (132a) und entsprechenden Armen (124b) (124a) des ersten Paars von Armen (124b) (124a) ein größter Winkel (142) ist, der erzielbar ist.

5. System (101) nach Anspruch 1, wobei jede der Elektroden (130a) (130b) (138a) (138b) einen jeweiligen geometrischen Mittelpunkt aufweist, und ein Winkel (142) zwischen den Elektroden (138a) (138b) des zweiten Paars und entsprechenden der Elektroden (406) (138b) (130a) (130b) des ersten Paars in einer ungedrehten Ausrichtung 63,4 Grad plus oder minus 0,5 Grad beträgt und in einer vollständige gedrehten Ausrichtung 88,5 Grad plus oder minus 0,5 Grad beträgt.

6. System (101) nach Anspruch 1, wobei die von dem ersten Paar von Armen (124b) (124a) getragenen Elektroden (130a) (130b) und die von dem zweiten Paar von Armen (132b) (132a) getragenen Elektroden (138a) (138b) jeweils eine raue freiliegende Oberfläche aufweisen.

7. System (101) nach Anspruch 1, weiter umfassend:
eine Schaltung (307), wobei die Schaltung (307) kommunikativ mit den von dem ersten Paar von Armen (124b) (124a) getragenen Elektroden (130a) (130b) und den von dem zweiten Paar von Armen (132b) (132a) getragenen Elektroden (138a) (138b) gekoppelt ist, wobei die Schaltung (307) betriebsfähig ist, um über die von dem ersten und dem zweiten Paar von Armen (124b) (124a) (132b) (132a) getragenen Elektroden (130a) (130b) (138a) (138b) einen Mikrostrom abzugeben, wobei die Schaltung (307) betriebsfähig ist, um Folgendes abzugeben: i) ein erstes Mikrostromsignal zwischen einer ersten der Elektroden des ersten Elektrodenpaars (130a) (130b) und einer ersten der Elektroden des zweiten Elektrodenpaars (138a) (138b), ii) ein zweites Mikrostromsignal zwischen einer zweiten der Elektroden des ersten Elektrodenpaars (130a) (130b) und einer zweiten der Elektroden des zweiten Elektrodenpaars (138a) (138b), ii) ein drittes Mikrostromsignal zwischen der ersten der Elektroden des ersten Elektrodenpaars (130a) (130b) und der zweiten der Elektroden des zweiten Elektrodenpaars (138a) (138b), iv) ein viertes Mikrostromsignal zwischen der zweiten der Elektroden des ersten Elektrodenpaars (130a) (130b) und der zweiten der Elektroden des zweiten Elektrodenpaars (138a) (138b).

8. System (101) nach Anspruch 7, wobei die Schaltung (307) betriebsfähig ist, um das erste Mikrostromsignal, das zweite Mikrostromsignal, das dritte Mikrostromsignal und das vierte Mikrostromsignal gleichzeitig abzugeben, und das erste Mikrostromsignal einen ersten Satz von Frequenzen aufweist, das zweite Mikrostromsignal einen zweiten Satz von Frequenzen aufweist, das dritte Mikrostromsignal einen dritten Satz von Frequenzen aufweist, und das vierte Mikrostromsignal einen vierten Satz von Frequenzen aufweist, wobei sich der zweite Satz von Frequenzen von dem ersten Satz von Frequenzen unterscheidet, der dritte Satz von Frequenzen sich von dem ersten und dem zweiten Satz von Frequenzen unterscheidet, und der vierte Satz von Frequenzen sich von dem ersten, dem zweiten und dem dritten Satz von Frequenzen unterscheidet.

9. System (101) nach Anspruch 7, wobei der Körper (102), das erste Paar von Armen (124b) (124a) und das zweite Paar von Armen (132b) (132a) eine tragbare Vorrichtung (500) (100) umfassen und die Schaltung (307) von dem Körper (102) der tragbaren Vorrichtung (500) (100) aufgenommen ist.

10. System (101) nach Anspruch 7, wobei der Körper (102), das erste Paar von Armen (124b) (124a) und das zweite Paar von Armen (132b) (132a) eine tragbare Vorrichtung (500) (100) umfassen, und weiter umfassend:
eine Konsole (502), an die der Körper (102) kommunikativ gekoppelt ist, wobei die Schaltung (307) von der Konsole (502) aufgenommen ist.

11. System (101) nach Anspruch 1, wobei der Körper (102) ein Greifkörper (102) ist, der Abmessungen aufweist, die bemessen sind, um in einer Hand eines Benutzers mit einer durchschnittlich bemessenen Hand gegriffen zu werden, und eine Benutzeroberfläche (506) (150), die von einer Außenseite (152) des Körpers (102) zugänglich ist.

12. System (101) nach Anspruch 1, wobei das erste Paar von Armen (124b) (124a) fest an dem Körper (102) gekoppelt sind und in Bezug auf den Körper (102) nicht schwenken, und weiter umfassend:
ein Paar von Abdichtungen, wobei jede der Abdichtungen ein jeweiliges Gelenk des ersten Paars von Gelenken (148), an dem ein jeweiliger der Arme (132b) (132a) des zweiten Paars von Armen (132b) (132a) schwenkbar an den Körper (102) gekoppelt ist, abdichtet.

## Revendications

1. Système (101), comprenant :
un corps (102) présentant une première extrémité (104) et une deuxième extrémité (106), la deuxième extrémité (106) étant espacée de la première extrémité (104) le long d'une longueur (108) du corps (102) ;
une première paire de bras (124b) (124a) qui s'étendent vers l'extérieur depuis la première extrémité (104) du corps (102), les bras (124b) (124a) de la première paire de bras (124b) (124a) étant espacés latéralement l'un de l'autre par rapport à une largeur (122) du corps (102), chacun des bras (124b) (124a) de la première paire de bras (124b) (124a) présentant une terminaison respective au niveau d'une extrémité distale (128b) (128a) du bras et chacun des bras (124b) (124a) de la première paire de bras (124b) (124a) présentant une électrode respective positionnée au moins à proximité de la terminaison respective du bras ;
une deuxième paire de bras (132b) (132a) qui s'étendent vers l'extérieur depuis la première extrémité (104) du corps (102), les bras (132b) (132a) de la deuxième paire de bras (132b) (132a) étant espacés latéralement l'un de l'autre par rapport à une largeur (122) du corps (102), chacun des bras (132b) (132a) de la deuxième paire de bras (132b) (132a) présentant une terminaison respective au niveau d'une extrémité distale (136b) (136a) du bras et chacun des bras (132b) (132a) de la deuxième paire de bras (132b) (132a) présentant une électrode respective positionnée au moins à proximité de la terminaison respective du bras, dans lequel les bras (132b) (132a) de la deuxième paire de bras (132b) (132a) peuvent pivoter par rapport aux bras (124b) (124a) de la première paire de bras (124b) (124a) pour faire varier un angle (142) entre les électrodes (138a) (130b) des bras (132b) (132a) de la deuxième paire de bras (132b) (132a) et les électrodes (130a) (130b) des bras (124b) (124a) de la première paire de bras (124b) (124a) ;
au moins une première paire d'articulations (148), les articulations de la première paire d'articulations accouplent respectivement de manière pivotante les bras respectifs des bras (132b) (132a) de la deuxième paire de bras (132b) (132a) au corps (102) au niveau d'un premier emplacement d'articulation (146) du corps (102) et d'un deuxième emplacement d'articulation (146) du corps (102) respectivement, le premier emplacement d'articulation (146) et le deuxième emplacement d'articulation (146) étant différents l'un de l'autre ; et
au moins un mécanisme de sollicitation (200) accouplé pour solliciter les bras (132b) (132a) de la deuxième paire de bras (132b) (132a) vers une orientation non tournée dans laquelle un angle (142) entre les bras (132b) (132a) de la deuxième paire de bras (132b) (132a) et les bras correspondants des bras (124b) (124a) de la première paire de bras (124b) (124a) est un angle le plus petit (142) réalisable entre ceux-ci.

2. Système (101) selon la revendication 1, dans lequel au moins un mécanisme de sollicitation (200) comprend au moins un ressort (202).

3. Système (101) selon la revendication 1, dans lequel au moins un mécanisme de sollicitation (200) applique une tension au tissu corporel lorsque les électrodes (130a) (130b) (138a) (138b) des première et deuxième paires de bras (124b) (124a) (132b) (132a) sont appliquées au tissu cutané pour obtenir un effet kinésiologique.

4. Système (101) selon la revendication 1, comprenant en outre :
une butée qui limite la rotation de la deuxième paire de bras (132b) (132a) par rapport à la première paire de bras (124b) (124a) à une orientation complètement tournée dans laquelle un angle (142) entre les bras (132b) (132a) de la deuxième paire de bras (132b) (132a) et les bras correspondants (124b) (124a) de la première paire de bras (124b) (124a) est un angle le plus grand (142) réalisable.

5. Système (101) selon la revendication 1 dans lequel chacune des électrodes (130a) (130b) (138a) (138b) présente un centre géométrique respectif et un angle (142) entre les électrodes (138a) (138b) de la deuxième paire et les électrodes correspondantes des électrodes (406) (138b) (130a) (130b) de la première paire est de 63,4 degrés plus ou moins 0,5 degré dans une orientation non tournée et est de 88,5 degrés plus ou moins 0,5 degré dans une orientation complètement tournée.

6. Système (101) selon la revendication 1 dans lequel les électrodes (130a) (130b) portées par la première paire de bras (124b) (124a) et les électrodes (138a) (138b) portées par la deuxième paire de bras (132b) (132a) présentent chacune une surface exposée rugueuse.

7. Système (101) selon la revendication 1, comprenant en outre :
un circuit (307), le circuit (307) étant accouplé de façon à communiquer avec les électrodes (130a) (130b) portées par la première paire de bras (124b) (124a) et les électrodes (138a) (138b) portées par la deuxième paire de bras (132b) (132a), le circuit (307) étant conçu pour émettre un microcourant par l'intermédiaire des électrodes (130a) (130b) (138a) (138b) portées par les première et deuxième paires de bras (124b) (124a) (132b) (132a), dans lequel le circuit (307) est conçu pour émettre : i) un premier signal de microcourant entre une première des électrodes de la première paire d'électrodes (130a) (130b) et une première des électrodes de la deuxième paire d'électrodes (138a) (138b), ii) un deuxième signal de microcourant entre une deuxième des électrodes de la première paire d'électrodes (130a) (130b) et une deuxième des électrodes de la deuxième paire d'électrodes (138a) (138b), iii) un troisième signal de microcourant entre la première des électrodes de la première paire d'électrodes (130a) (130b) et la deuxième des électrodes de la deuxième paire d'électrodes (138a) (138b), et iv) un quatrième signal de microcourant entre la deuxième des électrodes de la première paire d'électrodes (130a) (130b) et la deuxième des électrodes de la deuxième paire d'électrodes (138a) (138b).

8. Système (101) selon la revendication 7 dans lequel le circuit (307) est conçu pour émettre le premier signal de microcourant, le deuxième signal de microcourant, le troisième signal de microcourant et le quatrième signal de microcourant simultanément les uns aux autres, et le premier signal de microcourant présente un premier ensemble de fréquences, le deuxième signal de microcourant présente un deuxième ensemble de fréquences, le troisième signal de microcourant présente un troisième ensemble de fréquences, et le quatrième signal de microcourant présente un quatrième ensemble de fréquences, le deuxième ensemble de fréquences étant différent du premier ensemble de fréquences, le troisième ensemble de fréquences étant différent des premier et deuxième ensembles de fréquences ; et le quatrième ensemble de fréquences étant différent du premier, du deuxième et du troisième ensemble de fréquences.

9. Système (101) selon la revendication 7 dans lequel le corps (102), la première paire de bras (124b) (124a) et la deuxième paire de bras (132b) (132a) comprennent un dispositif portatif (500) (100) et le circuit (307) est logé par le corps (102) du dispositif portatif (500) (100).

10. Système (101) selon la revendication 7 dans lequel le corps (102), la première paire de bras (124b) (124a) et la deuxième paire de bras (132b) (132a) comprennent un dispositif portatif (500) (100), et comprenant en outre :
une console (502) à laquelle le corps (102) est accouplé de façon à communiquer avec celle-ci, dans lequel le circuit (307) est logé par la console (502).

11. Système (101) selon la revendication 1 dans lequel le corps (102) est un corps de poignée (102), présentant des dimensions ajustées pour être saisies dans une main d'un utilisateur présentant des mains de taille moyenne, et une interface utilisateur (506) (150) accessible depuis un extérieur (152) du corps (102).

12. Système (101) selon la revendication 1 dans lequel la première paire de bras (124b) (124a) est accouplée à demeure au corps (102) et ne pivote pas par rapport au corps (102), et comprenant en outre :
une paire de joints, chacun des joints scellant une articulation respective de la première paire d'articulations (148) au niveau de laquelle un bras respectif des bras (132b) (132a) de la deuxième paire de bras (132b) (132a) est accouplé de manière pivotante au corps (102).
